# EUROPÄISCHE PATENTSCHRIFT

(11) **EP 0 761 817 B1**
(45) Veröffentlichungstag und Bekanntmachung des Hinweises auf die Patenterteilung: **06.11.2002**
(21) Anmeldenummer: 96113289.1
(22) Anmeldetag: 20.08.1996
(51) Int. Cl.: C12P 7/24, C12N 1/20, C11B 9/00

(54) **Verfahren zur Herstellung von Vanillin und dafür geeignete Mikroorganismen**
Process for the preparation of vanillin and suitable microorganisms
Procédé de préparation de vanilline et les microorganismes appropriés

(30) Priorität: 01.09.1995 DE 19532317
(43) Veröffentlichungstag der Anmeldung: 12.03.1997
(73) Patentinhaber: HAARMANN & REIMER GMBH, D-37601 Holzminden (DE)
(72) Erfinder: Rabenhorst, Jürgen, Dr., 37671 Höxter (DE); Hopp, Rudolf, Dr., 37603 Holzminden (DE); Byng,Graham S., Dr., Snohomish, WA 98296 (US)
(74) Vertreter: Petrovicki, Wolfgang, Dr.

(56) Entgegenhaltungen:
- EP-A- 0 405 197
- WO-A-94/02621
- WO-A-96/08576
- US-A- 5 128 253
- SUTHERLAND J B ET AL: "METABOLISM OF CINNAMIC, P-COUMARIC, AND FERULIC ACIDS BY STREPTOMYCES SETONII" CANADIAN JOURNAL OF MICROBIOLOGY, Bd. 29, Nr. 10, 1. Januar 1983, Seiten 1253-1257, XP000650324

## Beschreibung

Die Erfindung betrifft ein fermentatives Verfahren zur Herstellung von Vanillin aus Ferulasäure und für dieses Verfahren geeignete Mikroorganismen.

Vanillin ist ein wichtiger, in der Nahrungs- und Genußmittel-Industrie viel verwendeter Aromastoff. Bislang wird es hauptsächlich aus dem Lignin der Sulfitablaugen, gelegentlich auch durch Oxidation von Eugenol oder Isoeugenol auf chemischem Wege hergestellt. Das auf diese Weise erhaltene Vanillin hat jedoch den Nachteil, daß es im lebensmittelrechtlichen Sinne kein Naturstoff ist und deshalb auch nicht als natürlicher Aromastoff bezeichnet werden darf.

Der natürliche Aromastoff Vanillin ist bisher nur durch Extraktion von Vanilleschoten erhältlich; das auf diese Weise erhaltene natürliche Vanillin ist jedoch sehr teuer. Natürliche Aromastoffe sind chemisch definierte Stoffe mit Aromaeigenschaften, gewonnen durch geeignete physikalische Verfahren (einschließlich Destillation und Extraktion mit Lösungsmitteln), durch enzymatische oder mikrobiologische Verfahren aus Ausgangsstoffen pflanzlicher oder tierischer Herkunft, die als solche verwendet oder mittels herkömmlicher Lebensmittelzubereitungsverfahren (einschließlich Trocknen, Rösten, Fermentieren) für den menschlichen Verzehr aufbereitet werden.

Verschiedene andere Verfahren zur Herstellung von natürlichem Vanillin mit unterschiedlichen Mikroorganismen und Enzymen sind in der Zwischenzeit veröffentlicht worden, liefern aber bislang aufgrund der nur recht geringen Ausbeuten bzw. Konzentrationen keine Produkte auf dem Markt; vgl. EP-A 405 197, 453 368 und 542 348, US-PS 5 128 253, JP-A 267 268 (25.10.1988) und 267 284 (25.10.1988).

In der wissenschaftlichen Literatur wird bei ganz verschiedenen Organismen das Auftreten von Vanillin beim Abbau von Ferulasäure als Intermediärprodukt beschrieben, aber in der Regel keine Konzentrationsangaben gemacht; vgl. S. Toms et al., "The Degradation of trans-Ferulic Acid by Pseudomonas acidovorans" in Biochemistry 9 (1970), 337-343; J.B. Sutherland et al., "Metabolism of Cinnamic, p-Coumaric and Ferulic Acid by Streptomyces setonii" in Can. J. Microbiol. 29 (1983), 1253-1257; G. Ötük, "Degradation of Ferulic Acid by Escherichia coli" in J. Ferment. Technol. 63 (1985), 501-506; S. Nazareth et al., "Degradation of Ferulic Acid via 4-Vinylguaiacol by Fusarium solani (Mart.) Sacc." in Can. J. Microbiol. 32 (1986) 494-497; M. Rahouti et al., "Metabolism of Ferulic Acid by Paecilomyces variotii and Pestalotia palmarum" in Appl. Environm. Microbiology 55 (1989), 2391-2398).

Teilweise sind die angegebenen Stämme auch nicht in Stammsammlungen hinterlegt und daher für Versuche anderer nicht zugänglich.

Auf der Suche nach einem neuen, praktisch anwendbaren Herstellungsverfahren für natürliches Vanillin aus günstig verfügbaren Ausgangsstoffen wurde jetzt überraschenderweise gefunden, daß dieses auf wirtschaftliche Weise mit Hilfe von bestimmten Bakterien aus der Gruppe der Actinomyceten in guten Ausbeuten und Konzentrationen durch Umsetzung von natürlicher Ferulasäure erhalten wird.

Gegenstand der Erfindung sind also neue Amycolatopsis species aus der Gattung Pseudonocardia mit den bei der Deutschen Sammlung für Mikroorganismen und Zellkulturen GmbH in Braunschweig unter den Nummern DSM 9991 und DSM 9992 hinterlegten Stämmen.

Aufgrund der chemotaxonomischen Untersuchungsergebnisse wurden beide Isolate der Gattung Amycolatopsis (Familie Pseudonocardiaceae) zugeordnet. Die Identifizierung basierte auf folgenden fünf Merkmalen:
1. Diagn. Aminosäure des Peptidoglycans: meso-Diaminopimelinsäure;
2. Diagn. Zucker aus Ganzzellhydrolysaten: Arabinose und Galaktose;
3. Mycolsäuren: fehlen;
4. Menachinone: MK - 9 (H₄);
5. Fettsäuremuster: Iso/Anteiso- + 10methyl-verzweigte gesättigte und ungesättigte Fettsäuren plus 2-Hydroxy-Fettsäuren. Dieses Fettsäuremuster ist diagnostisch für Vertreter der Gattung Amycolatopsis.

Da DSM 9991 und DSM 9992 auch das typische Erscheinungsbild der Vertreter der Gattung Amycolatopsis zeigen - beige bis gelbes Substratmycel und auf einigen Medien auch ein feines weißes Luftmycel - wird die Zuordnung von DSM 9991 und DSM 9992 zur Gattung Amycolatopsis zusätzlich durch die Morphologie unterstützt.

Der Vergleich der Fettsäuremuster der beiden Stämme mit den Einträgen der DSM-Fettsäure-Datenbanken durch eine Hauptkomponentenanalyse ergab für beide Stämme eine Zuordnung zu Amycolatopsis mediterranei. Der Similaritätsindex ist aber in beiden Fällen zu niedrig (0,037 und 0,006), um eine definitive Spezies-Zuordnung zuzulassen.

Zusätzlich durchgeführte Analysen der 16S rDNA und der Vergleich der diagnostischen Partialsequenzen mit den 16S rDNA Datenbandeinträgen von Amycolatopsis Typus Stämmen, unterstützen die Ergebnisse der Fettsäureuntersuchungen. Auch bei den 16S rDNA Sequenzen konnte keine hohe Homologie zu den bekannten Amycolatopsis Typus Stämmen nachgewiesen werden. Im Gegensatz zu den Fettsäure-Ergebnissen, bei denen keine Unterschiede in den Mustern der beiden Isolate gefunden wurden, war es mithilfe der 16S rDNA Sequenzen möglich, DSM 9991 und DSM 9992 zu differenzieren. Aufgrund der großen Unterschiede in den 16S rDNA Sequenzen (Similarität 99,6 %), kann davon ausgegangen werden, daß DSM 9991 und DSM 9992 Stämme von jeweils eigenständigen bisher unbeschriebenen Amycolatopsis Spezies sind.

Weiterer Gegenstand der Erfindung ist ein Verfahren zur Herstellung von Vanillin aus Ferulasäure in Gegenwart von neuen Amycolatopsis sp.

Weiterer Gegenstand der Erfindung ist die Verwendung des so hergestellten Vanillins zur Herstellung von Aromen.

Die als Ausgangsmaterial bevorzugte natürliche Ferulasäure kann unter anderem aus natürlichem Eugenol durch Umsetzung mit Pseudomonas sp. DSM 7062 oder DSM 7063 erhalten werden (DE-OS 4 227 076).

Der Organismus kann in einem üblichen Kulturmedium in für die Kultivierung von Mikroorganismen üblichen Weise kultiviert werden. Das Substrat kann zu Beginn der Inkubation, während oder nach Abschluß des Wachstums auf einmal oder über einen längeren Zeitraum verteilt zugegeben werden. Die Menge an Ferulasäure wird dabei vorteilhaft so bemessen, daß die Konzentration der Verbindung in der Kulturbrühe 80 g/l, vorzugsweise 15 g/l nicht überschreitet. Der Verlauf der Umsetzung kann durch Bestimmung des Ausgangsmaterials und des Produkts in der Kulturbrühe mittels Hochdruckflüssigkeitschromatographie verfolgt werden. Nachdem die optimale Menge an Vanillin entstanden ist, wird dieses durch bekannte physikalische Verfahren wie Extraktion, Destillation oder Chromatographie aus der Kulturbrühe isoliert. Das so erhaltene Rohprodukt kann durch weitere Schritte gereinigt werden.

Die Kultivierung des erfindungsgemäßen Mikroorganismus kann in synthetischen, halbsynthetischen oder komplexen Kulturmedien erfolgen. Diese Kulturmedien enthalten Kohlenstoffquellen, Stickstoffquellen, anorganische Salze und gegebenenfalls Spurenelemente und Vitamine.

Als Kohlenstoffquellen können z.B. Zucker wie Glucose, Zuckeralkohole wie Glycerin oder Mannit, organische Säuren wie Zitronensäure oder komplexe Gemische wie Malzextrakt, Hefeextrakt, Casein oder Caseinhydrolysat dienen.

Beispiele für geeignete Stickstoffquellen sind anorganische Stickstoffquellen wie Nitrate und Ammoniumsalze und organische Stickstoffquellen wie Hefeextrakt, Sojamehl, Baumwollsaatmehl, Casein, Caseinhydrolysat, Weizengluten und Maisquellwasser.

Als anorganische Salze können beispielsweise unter anderem Sulfate, Nitrate, Chloride, Carbonate und Phosphate von Natrium, Kalium, Magnesium, Calcium, Zink und Eisen verwendet werden.

Die Kultivierungstemperatur liegt vorzugsweise im Bereich von 10 bis 55°C, besonders bevorzugt im Bereich von 35 bis 45°C. Der pH-Wert des Mediums beträgt bevorzugt 3 bis 9, insbesondere 4 bis 8. Die Kultivierung kann entweder in geeigneten Schüttelapparaturen oder in Fermentern mit Mischeinrichtung erfolgen. Bei der Kultivierung ist für eine ausreichende Belüftung Sorge zu tragen. Die Kultivierung kann batchweise, halbkontinuierlich oder kontinuierlich durchgeführtwerden. Die Kulturdauer bis zum Erreichen einer maximalen Produktmenge liegt zwischen 4 und 120 Stunden nach dem Animpfen der Kultur. Um die Mikroorganismen vor der toxischen Wirkung der eingesetzten bzw. gebildeten Substanzen zu schützen, kann es vorteilhaft sein, den Kulturmedien Adsorptionsmittel zuzusetzen, z.B. Aktivkohle oder Adsorberharze wie ®Amberlite XAD-2, ®Amberlite XAD-7, XAD-16, ®Lewatit OC 1062 oder OC 1064.

Die Prozentangaben der nachfolgenden Beispiele beziehen sich jeweils auf das Gewicht.

### Beispiele

### Beispiel 1

### Herstellung der Vorkultur

Ein 500 ml Erlenmeyerkolben mit seitlichem Einstich wurde mit 100 ml Medium, bestehend aus 1 g Malzextrakt, 0,4 g Glucose und 0,4 g Hefeextrakt ad 100 ml Wasser, gefüllt und anschließend 20 Minuten bei 121°C sterilisiert. Nach dem Abkühlen wurde der Kolben mit 200 µl einer eingefrorenen Glycerinkultur Amycolatopsis sp. DSM 9991 oder DSM 9992 beimpft. Die Kultur wurde auf einer rotierenden Schüttelmaschine bei 45°C und 100 Upm inkubiert. Nach 24 Stunden wurde diese Kultur zur Beimpfung des Produktionsmediums verwendet.

### Beispiel 2

### Produktion im Schüttelkolben

Acht 500 ml Erlenmeyerkolben mit seitlichem Einstich wurden mit je 100 ml Medium (4 g/l Glucose, 10 g/l Malzextrakt und 4 g/l Hefeextrakt) gefüllt und anschließend 20 Minuten bei 121°C dampfsterilisiert.

Nach dem Abkühlen wurden die Kolben mit je 2 ml einer Kultur von Amycolatopsis sp. DSM 9992 aus Beispiel 1 angeimpft. Die Kulturen wurden auf einer rotierenden Schüttelmaschine bei 37°C und 100 Upm inkubiert.

16 Stunden nach dem Animpfen wurden 20 ml, nach 24 Stunden 40 ml und nach 44 Stunden nochmals 10 ml einer 3,3%igen sterilfiltrierten Ferulasäurelösung zu jedem Kolben zugegeben. Nach 47 Stunden wurde die Kulturbrühe der Kolben vereinigt und der Gehalt an Vanillin und nicht umgesetzter Ferulasäure bestimmt.

Der Gehalt an Vanillin in der Kulturbrühe lag gemäß HPLC-Analysen bei 7317 ppm. 1526 ppm Ferulasäure waren noch nicht umsetzt. Das Endgewicht der Kultur der 8 Kolben betrug 1420 g. Dies ist eine Umsetzung von ca. 72 % der Theorie, bezogen auf die umgesetzte Ferulasäure.

### Beispiel 3

### Produktion von Vanillin im 10 l Fermenter

5 l Kulturmedium (4 g/l Glucose, 10 g/l Malzextrakt und 6 g/l Hefeextrakt) wurden in einem Fermenter sterilisiert und nach Abkühlen mit 100 ml einer Vorkultur von DSM 9992 gemäß Beispiel 1 angeimpft.

Die Kulturbedingungen waren: 37°C, 500 Upm, 5 l Luft/Min. 12,5 Stunden nach dem Animpfen wurden 1,634 kg einer ca. 3,7%igen Ferulasäurelösung (60,2 g Ferulasäure) zugegeben. Nach 17,5 Stunden wurden über einen Zeitraum von 10 Stunden weitere 4,397 kg einer ca. 3,7%igen Ferulasäurelösung (164,72 g Ferulasäure) zugepumpt.

Nach 32 Stunden wurde die Fermentation abgebrochen. Die Konzentration an Vanillin lag bei 11,5 g/l und es lag noch 1 g/l nicht umgesetzte Ferulasäure vor. Das Endvolumen betrug 11,29 l. Dies ist eine Umsetzung von 77,8 % der Theorie, bezogen auf umgesetzte Ferulasäure.

## Patentansprüche

1. Amycolatopsis sp. DSM 9991

2. Verfahren zur Herstellung von Vanillin aus Ferulasäure in Gegenwart von Amycolatopsis sp. DSM 9991.

3. Verfahren gemäß Anspruch 2, **dadurch gekennzeichnet, dass** Amycolatopsis sp. DSM 9991 in einem Kulturmedium kultiviert wird, anschließend Ferulasäure zum Kulturmedium zugesetzt wird und nach Beendigung der Umsetzung Vanillin isoliert wird.

4. Verfahren gemäß einem oder mehreren der vorangegangenen Ansprüche 2 und 3, **dadurch gekennzeichnet, dass** die Konzentration der Ferulasäure im Kulturmedium 80 g/l nicht überschreitet.

5. Verfahren gemäß einem oder mehreren der vorangegangenen Ansprüche 2 bis 4, **dadurch gekennzeichnet, dass** die Konzentration der Ferulasäure im Kulturmedium 15 g/l nicht überschreitet.

6. Verfahren gemäß einem oder mehreren der vorangegangenen Ansprüche 2 bis 5, **dadurch gekennzeichnet, dass** Ferulasäure nicht auf einmal, sondern in mehreren Portionen zum Kulturmedium zugesetzt wird.

7. Verfahren gemäß einem oder mehreren der vorangegangenen Ansprüche 2 bis 6, **dadurch gekennzeichnet, dass** das Kulturmedium mindestens eine Kohtenstoffquelle, eine Stickstoffquelle, anorganische Salze und gegebenenfalls Spurenelemente und Vitamine enthält.

8. Verfahren gemäß einem oder mehreren der vorangegangenen Ansprüche 2 bis 7, **dadurch gekennzeichnet, dass** das Kulturmedium als Kohlenstoffquelle Zucker, Zuckeralkohole, organische Säuren oder komplexe Gemische enthält.

9. Verfahren gemäß einem oder mehreren der vorangegangenen Ansprüche 2 bis 8, **dadurch gekennzeichnet, dass** das Kulturmedium als Kohlenstoffquelle Glucose, Malzextrakt und Hefeextrakt enthält.

10. Verfahren gemäß einem oder mehreren der vorangegangenen Ansprüche 2 bis 9, **dadurch gekennzeichnet, dass** das Kulturmedium als Stickstoffquelle anorganische Stickstoffquellen oder organische Stickstoffquellen enthält.

11. Verfahren gemäß einem oder mehreren der vorangegangenen Ansprüche 2 bis 10, **dadurch gekennzeichnet, dass** das Kulturmedium als Stickstoffquelle Hefeextrakt enthält.

12. Verfahren zur Herstellung von Vanillin durch
(a) Kultivierung von Amycolatopsis sp. DSM 9991 in einem Kulturmedium enthaltend Hefeextrakt, Malzextrakt und Glucose
(b) Zusatz von Ferulasäure, wobei Ferulasäure nicht auf einmal, sondern in mehreren Portionen zugesetzt wird und die Konzentration der Ferulasäure im Kulturmedium 80 g/l nicht überschreitet
(c) Isolierung des Vanillins nach Beendigung der Umsetzung.

13. Verfahren gemäß einem oder mehreren der vorangegangenen Ansprüche 2 bis 12, **dadurch gekennzeichnet, dass** natürliche Ferulasäure eingesetzt wird.

14. Verfahren zur Herstellung von Aromen, **dadurch gekennzeichnet, dass**
(a) Vanillin aus Ferulasäure in Gegenwart von Amycolatopsis sp. DSM 9991 hergestellt wird und
(b) das in Schritt (a) hergestellte Vanillin zur Herstellung von Aromen verwendet wird.

15. Amycolatopsis sp. DSM 9992.

16. Verfahren zur Herstellung von Vanillin aus Ferulasäure in Gegenwart von Amycolatopsis sp. DSM 9992.

17. Verfahren gemäß Anspruch 16, **dadurch gekennzeichnet, dass** Amycolatopsis sp. DSM 9992 in einem Kulturmedium kultiviert wird, anschließend Ferulasäure zum Kulturmedium zugesetzt wird und nach Beendigung der Umsetzung Vanillin isoliert wird.

18. Verfahren gemäß einem oder mehreren der vorangegangenen Ansprüche 16 und 17, **dadurch gekennzeichnet, dass** die Konzentration der Ferulasäure im Kulturmedium 80 g/l nicht überschreitet.

19. Verfahren gemäß einem oder mehreren der vorangegangenen Ansprüche 16 bis 18, **dadurch gekennzeichnet, dass** die Konzentration der Ferulasäure im Kulturmedium 15 g/l nicht überschreitet.

20. Verfahren gemäß einem oder mehreren der vorangegangenen Ansprüche 16 bis 19, **dadurch gekennzeichnet, dass** Ferulasäure nicht auf einmal, sondern in mehreren Portionen zum Kulturmedium zugesetzt wird.

21. Verfahren gemäß einem oder mehreren der vorangegangenen Ansprüche 16 bis 20, **dadurch gekennzeichnet, dass** das Kulturmedium mindestens eine Kohlenstoffquelle, eine Stickstoffquelle, anorganische Salze und gegebenenfalls Spurenelemente und Vitamine enthält.

22. Verfahren gemäß einem oder mehreren der vorangegangenen Ansprüche 16 bis 20, **dadurch gekennzeichnet, dass** das Kulturmedium als Kohlenstoffquelle Zucker, Zuckeralkohole, organische Säuren oder komplexe Gemische enthält.

23. Verfahren gemäß einem oder mehreren der vorangegangenen Ansprüche 16 bis 22, **dadurch gekennzeichnet, dass** das Kulturmedium als Kohlenstoffquelle Glucose, Malzextrakt und Hefeextrakt enthält.

24. Verfahren gemäß einem oder mehreren der vorangegangenen Ansprüche 16 bis 23, **dadurch gekennzeichnet, dass** das Kulturmedium als Stickstoffquelle anorganische Stickstoffquellen oder organische Stickstoffquellen enthält.

25. Verfahren gemäß einem oder mehreren der vorangegangenen Ansprüche 16 bis 24, **dadurch gekennzeichnet, dass** das Kulturmedium als Stickstoffquelle Hefeextrakt enthält.

26. Verfahren zur Herstellung von Vanillin durch
(a) Kultivierung von Amycolatopsis sp. DSM 9992 in einem Kulturmedium enthaltend Hefeextrakt, Malzextrakt und Glucose
(b) Zusatz von Ferulasäure, wobei Ferulasäure nicht auf einmal, sondern in mehreren Portionen zugesetzt wird und die Konzentration der Ferulasäure im Kulturmedium 80 g/l nicht überschreitet
(c) Isolierung des Vanillins nach Beendigung der Umsetzung.

27. Verfahren gemäß einem oder mehreren der vorangegangenen Ansprüche 16 bis 26, **dadurch gekennzeichnet, dass** natürliche Ferulasäure eingesetzt wird.

28. Verfahren zur Herstellung von Aromen, **dadurch gekennzeichnet, dass**
(a) Vanillin aus Ferulasäure in Gegenwart von Amycolatopsis sp. DSM 9992 hergestellt wird und
(b) das in Schritt (a) hergestellte Vanillin zur Herstellung von Aromen verwendet wird.

## Claims

1. Amycolatopsis sp. DSM 9991.

2. Process for the preparation of vanillin from ferulic acid in the presence of amycolatopsis sp. DSM 9991.

3. Process according to claim 2, **characterised in that** amycolatopsis sp. DSM 9991 is cultivated in a culture medium, following which ferulic acid is added to the culture medium and vanillin is isolated after completion of the reaction.

4. Process according to one or both of the preceding claims 2 and 3, **characterised in that** the concentration of ferulic acid in the culture medium does not exceed 80 g/l

5. Process according to one or more of the preceding claims 2 to 4, **characterised in that** the concentration of ferulic acid in the culture medium does not exceed 15 g/l.

6. Process according to one or more of the preceding claims 2 to 5, **characterised in that** ferulic acid is added not in one portion but in several portions to the culture medium.

7. Process according to one or more of the preceding claims 2 to 6, **characterised in that** the culture medium contains at least one carbon source, a nitrogen source, inorganic salts and optionally trace elements and vitamins.

8. Process according to one or more of the preceding claims 2 to 7, **characterised in that** the culture medium contains as carbon source sugar, sugar alcohols, organic acids or complex mixtures.

9. Process according to one or more of the preceding claims 2 to 8, **characterised in that** the culture medium contains as carbon source glucose, malt extract and yeast extract.

10. Process according to one or more of the preceding claims 2 to 9, **characterised in that** the culture medium contains as nitrogen source inorganic nitrogen sources or organic nitrogen sources.

11. Process according to one or more of the preceding claims 2 to 10, **characterised in that** the culture medium contains as nitrogen source yeast extract.

12. Process for the preparation of vanillin by
(a) cultivation of amycolatopsis sp. DSM 9991 in a culture medium containing yeast extract, malt extract and glucose
(b) addition of ferulic acid, wherein ferulic acid is added not in one portion but in several portions and the concentration of ferulic acid in the culture medium does not exceed 80 g/l
(c) isolation of the vanillin after completion of the reaction.

13. Process according to one or more of the preceding claims 2 to 12, **characterised in that** natural ferulic acid is used.

14. Process for the production of flavouring agents, **characterised in that**
(a) vanillin is prepared from ferulic acid in the presence of amycolatopsis sp. DSM 9991, and
(b) the vanillin prepared in step (a) is used for the production of flavouring agents.

15. Amycolatopsis sp. DSM 9992.

16. Process for the preparation of vanillin from ferulic acid in the presence of amycolatopsis sp. DSM 9992.

17. Process according to claim 16, **characterised in that** amycolatopsis sp. DSM 9992 is cultivated in a culture medium, following which ferulic acid is added to the culture medium and vanillin is isolated after completion of the reaction.

18. Process according to one or both of the preceding claims 16 and 17, **characterised in that** the concentration of ferulic acid in the culture medium does not exceed 80 g/l.

19. Process according to one or more of the preceding claims 16 to 18, **characterised in that** the concentration of ferulic acid in the culture medium does not exceed 15 g/l.

20. Process according to one or more of the preceding claims 16 to 19, **characterised in that** ferulic acid is added not in one portion but in several portions to the culture medium.

21. Process according to one or more of the preceding claims 16 to 20, **characterised in that** the culture medium contains at least one carbon source, a nitrogen source, inorganic salts and optionally trace elements and vitamins.

22. Process according to one or more of the preceding claims 16 to 20, **characterised in that** the culture medium contains as carbon source sugar, sugar alcohols, organic acids or complex mixtures.

23. Process according to one or more of the preceding claims 16 to 22, **characterised in that** the culture medium contains as carbon source glucose, malt extract and yeast extract.

24. Process according to one or more of the preceding claims 16 to 23, **characterised in that** the culture medium contains as nitrogen source inorganic nitrogen sources or organic nitrogen sources.

25. Process according to one or more of the preceding claims 16 to 24, **characterised in that** the culture medium contains as nitrogen source yeast extract.

26. Process for the preparation of vanillin by
(a) cultivation of amycolatopsis sp. DSM 9992 in a culture medium containing yeast extract, malt extract and glucose
(b) addition of ferulic acid, wherein ferulic acid is added not in one portion but in several portions and the concentration of ferulic acid in the culture medium does not exceed 80 g/l
(c) isolation of the vanillin after completion of the reaction.

27. Process according to one or more of the preceding claims 16 to 26, **characterised in that** natural ferulic acid is used.

28. Process for the production of flavouring agents, **characterised in that**
(a) vanillin is prepared from ferulic acid in the presence of amycolatopsis sp. DSM 9992, and
(b) the vanillin prepared in step (a) is used for the production of flavouring agents.

## Revendications

1. Amycolatopsis sp. DSM 9991.

2. Procédé de production de vanilline à partir d'acide férulique en présence d'Amycolatopsis SP. DSM 9991.

3. Procédé selon la revendication 2, **caractérisé en ce que** Amycolatopsis sp. DSM 9991 est cultivé dans un milieu de culture, après quoi de l'acide férulique est ajouté au milieu de culture et la vanilline est isolée après la fin de la réaction.

4. Procédé selon une ou plusieurs des revendications 2 et 3 précédentes, **caractérisé en ce que** la concentration de l'acide férulique dans le milieu de culture ne dépasse pas 80 g/l.

5. Procédé selon une ou plusieurs des revendications 2 à 4 précédentes, **caractérisé en ce que** la concentration de l'acide férulique dans le milieu de culture ne dépasse pas 15 g/l.

6. Procédé selon une ou plusieurs des revendications 2 à 5 précédentes, **caractérisé en ce que** l'acide férulique n'est pas ajouté au milieu de culture en une fois, mais en plusieurs portions.

7. Procédé selon une ou plusieurs des revendications 2 à 6 précédentes, **caractérisé en ce que** le milieu de culture contient au moins une source de carbone, une source d'azote, des sels inorganiques et éventuellement des éléments à l'état de traces et des vitamines.

8. Procédé selon une ou plusieurs des revendications 2 à 7 précédentes, **caractérisé en ce que** le milieu de culture contient comme source de carbone des sucres, des alcools de sucres, des acides organiques ou des mélanges complexes.

9. Procédé selon une ou plusieurs des revendications 2 à 8 précédentes, **caractérisé en ce que** le milieu de culture contient comme source de carbone du glucose, de l'extrait de malt et de l'extrait de levure.

10. Procédé selon une ou plusieurs des revendications 2 à 9 précédentes, **caractérisé en ce que** le milieu de culture contient comme source d'azote des sources d'azote inorganiques ou des sources d'azote organiques.

11. Procédé selon une ou plusieurs des revendications 2 à 10 précédentes, **caractérisé en ce que** le milieu de culture contient de l'extrait de levure comme source d'azote.

12. Procédé de production de vanilline par
a) culture d'Amycolatopsis sp. DSM 9991 dans un milieu de culture contenant de l'extrait de levure, de l'extrait de malt et du glucose
b) addition d'acide férulique, l'acide férulique n'étant pas ajouté en une fois mais en plusieurs portions et la concentration de l'acide férulique dans le milieu de culture ne dépassant pas 80 g/l
c) isolement de la vanilline après la fin de la réaction.

13. Procédé selon une ou plusieurs des revendications 2 à 12 précédentes, **caractérisé en ce que** l'on utilise de l'acide férulique naturel.

14. Procédé de production d'arômes **caractérisé en ce que**
a) de la vanilline est préparée à partir d'acide férulique en présence d'Amycolatopsis sp. DSM 9991 et
b) la vanilline produite dans l'étape a) est utilisée pour la production d'arômes.

15. Amycolatopsis sp. DSM 9992.

16. Procédé de production de vanilline à partir d'acide férulique en présence d'Amycolatopsis SP. DSM 9992.

17. Procédé selon la revendication 16, **caractérisé en ce que** Amycolatopsis sp. DSM 9992 est cultivé dans un milieu de culture, après quoi de l'acide férulique est ajouté au milieu de culture et la vanilline est isolée après la fin de la réaction.

18. Procédé selon une ou plusieurs des revendications 16 et 17 précédentes, **caractérisé en ce que** la concentration de l'acide férulique dans le milieu de culture ne dépasse pas 80 g/l.

19. Procédé selon une ou plusieurs des revendications 16 à 18 précédentes, **caractérisé en ce que** la concentration de l'acide férulique dans le milieu de culture ne dépasse pas 15 g/l.

20. Procédé selon une ou plusieurs des revendications 16 à 19 précédentes, **caractérisé en ce que** l'acide férulique n'est pas ajouté au milieu de culture en une fois, mais en plusieurs portions.

21. Procédé selon une ou plusieurs des revendications 16 à 20 précédentes, **caractérisé en ce que** le milieu de culture contient au moins une source de carbone, une source d'azote, des sels inorganiques et éventuellement des éléments à l'état de traces et des vitamines.

22. Procédé selon une ou plusieurs des revendications 16 à 20 précédentes, **caractérisé en ce que** le milieu de culture contient comme source de carbone des sucres, des alcools de sucres, des acides organiques ou des mélanges complexes.

23. Procédé selon une ou plusieurs des revendications 16 à 22 précédentes, **caractérisé en ce que** le milieu de culture contient comme source de carbone du glucose, de l'extrait de malt et de l'extrait de levure.

24. Procédé selon une ou plusieurs des revendications 16 à 23 précédentes, **caractérisé en ce que** le milieu de culture contient comme source d'azote des sources d'azote inorganiques ou des sources d'azote organiques.

25. Procédé selon une ou plusieurs des revendications 16 à 24 précédentes, **caractérisé en ce que** le milieu de culture contient de l'extrait de levure comme source d'azote.

26. Procédé de production de vanilline par
a) culture d'Amycolatopsis sp. DSM 9992 dans un milieu de culture contenant de l'extrait de levure, de l'extrait de malt et du glucose
b) addition d'acide férulique, l'acide férulique n'étant pas ajouté en une fois mais en plusieurs portions et la concentration de l'acide férulique dans le milieu de culture ne dépassant pas 80 g/l
c) isolement de la vanilline après la fin de la réaction.

27. Procédé selon une ou plusieurs des revendications 16 à 26 précédentes, **caractérisé en ce que** l'on utilise de l'acide férulique naturel.

28. Procédé de production d'arômes **caractérisé en ce que**
a) de la vanilline est préparée à partir d'acide férulique en présence d'Amycolatopsis sp. DSM 9992 et
b) la vanilline produite dans l'étape a) est utilisée pour la production d'arômes.
